# EUROPEAN PATENT APPLICATION

(11) **EP 2 676 954 A1**
(43) Date of publication of application: **25.12.2013**
(21) Application number: 12384006.8
(22) Date of filing: 19.06.2012
(51) Int. Cl.: C07D 231/12, A61K 31/415, A61P 9/00, A61P 15/00

(54) **Heterocyclyl-substituted-phenyl derivatives as vasodilators**

(71) Applicant: Laboratorios del Dr. Esteve S.A., 08041 Barcelona (ES)
(72) Inventor: Romero-Alonso, Mª Luz, 08904 L'Hospilatet de Llobregat (Barcelona) (ES); Vela-Hernandez, Jose-Miguel, 08028 Barcelona (ES); Codony-Soler, Xavier, ES 08301 Mataro (Barcelona) (ES); Del Riego-Cuesta, Marta, 08041 Barcelona (ES)
(74) Representative: Ruiz, Nicolas Vincent

(57) **Abstract**

The present invention relates to heterocyclyl-substituted-phenyl compounds of general formula (I), for use as vasodilator agents and the pharmaceutical compositions comprising them. 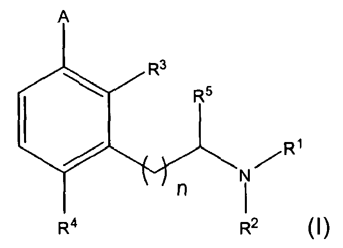 wherein A is a compound selected from the following group: 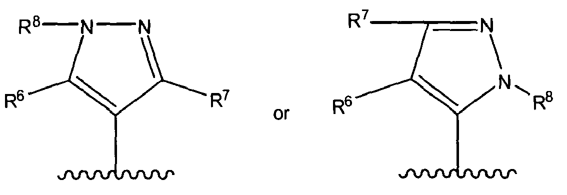 n is selected from 0 or 1.

## Description

### FIELD OF THE INVENTION

The present invention relates to heterocyclyl-substituted-phenyl derivatives, in particular, to their use as vasodilators and to the pharmaceutical compositions comprising them.

### BACKGROUND OF THE INVENTION

The 5-HT7 receptor was discovered in 1993. It belongs to the family of 5-hydroxytryptamine (serotonin, 5-HT) receptors and it has attracted great interest as a valuable new drug target (Terrón, J.A. Idrugs, 1998, vol. 1, no. 3, pages 302-310: "The 5HT7 receptor: A target for novel therapeutic avenues?").

5-HT7 receptors have been cioned from rat, mouse, guinea pig and human cDNA and exhibit a high degree of interspecies homology (approx. 95%), but it is unique in that it has a low sequence homology with other 5-HT receptors (less than 40%). Its expression pattern, in particular structures of the central nervous system (CNS) (highest in hypothalamus, in particular suprachiasmatic nuclei and thalamus) and other peripheral tissues (spleen, kidney, intestinal, heart and coronary artery), implicates the 5-HT7 receptor in a variety of functions and pathologies.

Patent application WO 2004/106934 deals with diagnostics and therapeutics for diseases associated with 5-HT7 receptor. This receptor was found to be expressed in various human tissues and, consequently, several therapeutic indications could be envisaged to be related with 5-HT7 receptor ligands. Among these therapeutic indications, of particular relevance are those in connection with the central and the peripheral nervous systems, such as CNS disorders (such as all types of pain, cerebrovascular diseases, Parkinson's disease, Alzheimer's disease, and the like), cardiovascular disorders and peripheral vascular diseases.

Some heterocyclyl-substituted-phenyl derivatives showing selective agonist activity towards 5-HT7 receptors were described in applications WO-2008/077625, WO-2008/095689, WO-2009/003719 and WO-2011/042302.

Patent application WO 2008/00495 studies and demonstrates the use of 5-HT7 receptor agonists for the manufacture of a medicament for the treatment of pain, especially, certain subtypes of pain like neuropathic pain and inflammatory pain and symptoms involving allodynia and hyperalgesia, as well as for the prevention or the prophylaxis of pain and the symptoms of pain, especially certain subtypes of pain like neuropathic pain and inflammatory pain and symptoms involving allodynia and hyperalgesia.

Distribution and early pharmacological data also suggest that the 5-HT7 receptor is involved in the vasodilatation of blood vessels. This has been demonstrated in vivo (Terrón, J.A., Br J Pharmacol, 1997, 121:563-571 "Role of 5-HT7 receptors in the long lasting hypotensive response induced by 5-hydroxytryptamine in the rat").

Several clinical conditions presenting vasoconstriction are treated with vasodilators. For instance, patients suffering from pulmonary hypertension are treated with nonselective entothelin receptor antagonists like bosentan or with phosphodiesterease 5 inhibitors like sildenafil or with prostacyclin analogues like iloprost. The management of hypertension of patients suffering from peripheral vascular disease is also treated with vasodilator agents like prostacyclin analogues or beta-adrenoceptor antagonists or phosphodiesterase inhibitors or phosphodiesterase 5 inhibitors.

Erectile Dysfunction (ED) is the inability to get or keep an erection firm enough for sexual intercourse. ED can be a total inability to achieve an erection, an inconsistent ability to do so, or a tendency to sustain only brief erections. ED has been estimated to affect 150 million individuals worldwide and data from ENIGMA study in 2004 suggested that this condition is prevalent in approximately 17% of all European men (Lasker, G. F. et al Advances in Pharmacological Sciences, 2010, "A review of the Pathophysiology and Novel treatments for Erectile Dysfunction"). It is estimated that the incidence of impotence is about 5% at 40 years of age and 15% to 25% at 65 years and above. The current standard of care for ED consists of lifestyle changes such as diet having direct impact on concomitant diseases like diabetes, hypertension and weight loss, along with pharmacotherapies. Drugs for treating ED can be taken orally, injected directly into the penis, or inserted into the urethra at the tip of the penis.

The current gold standard treatment is the use of phosphodiesterase 5 (PDE-5) inhibitors such as sildenafil citrate, vardenafil hydrochloride and tadalafil. Despite the successful use of PDE-5 inhibitors, there is still a significant population of patients that remain refractory to this therapy. ED in individuals with chronic disease states such as diabetes mellitus and cardiovascular disease often remains refractory to standard treatment with PDE-5.

Alternatively, several formulations comprising alprostadil have been developed for the treatment of ED. Alprostadil is a synthetic form of prostaglandin E1 and a smooth muscle relaxant which induces erection by relaxation of trabecular smooth muscle and by dilation of cavernous arteries.

The worldwide prevalence of ED in society requires new treatments for the growing number of patients with comorbid conditions that have become refractory to typical PDE-5 therapy.

In light of these facts, the applicant has now found that some selective 5-HT7 receptor agonists may be of a great value as vasodilator agents. This is in particular true for some heterocyclyl-substituted-phenyl derivatives. In particular, the vasodilator effect of the said selective 5-HT7 receptor agonists could be of great interest in the treatment of clinical conditions presenting vasoconstriction, including hypertension, pulmonary hypertension, peripheral vascular disease and ED.

### FIGURES

Figure 1: Effects of 5-HT7 receptor agonists according to the present invention on peripheral vasodilatation in conscious rats.
Figure 2: Effects of 5-HT7 receptor agonists according to the present invention on penile erection in conscious rats.

### BRIEF DESCRIPTION OF THE INVENTION

The present invention relates to the vasodilator activity of some selective 5-HT7 agonists . In particular, these compounds show a peripheral vasodilatation.

Thus, it is an aspect of the present invention to provide heterocyclyl substituted-phenyl derivatives of a general formula (I) for use as vasodilator agents: 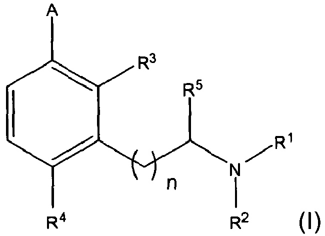 wherein the meanings of A, n, R¹, R², R³, R⁴ and R⁵ are defined below in section "detailed description of the invention".

A preferred embodiment of the present invention is to provide heterocyclyl substituted-phenyl derivatives of a general formula (Ia) for use as vasodilator agents: 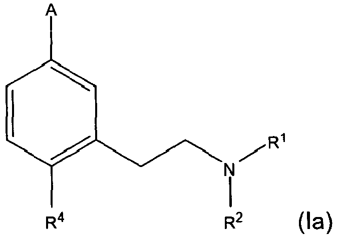 wherein the meanings of A, R¹, R² and R⁴ are defined below in section "detailed description of the invention".

In a most preferred embodiment, the invention relates to compounds of formula (la) for use as vasodilator agents wherein group A is 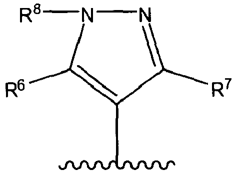 wherein the meanings of R⁶, R⁷ and R⁸ are defined below in section "detailed description of the invention".

In a further aspect, the invention relates to the said derivatives for the treatment of erectile dysfunction.

In another aspect, the invention relates to the said derivatives for the treatment of clinical conditions presenting vasoconstriction.

In another aspect, the invention relates to the said derivatives for the treatment of hypertension.

In another aspect, the invention relates to the said derivatives for the treatment of peripheral vascular disease.

In another aspect, the invention relates to the said derivatives for the treatment of pulmonary arterial hypertension.

In other aspect, the invention deals with pharmaceutical composition comprising a therapeutically effective amount of the said derivatives, together with pharmaceutically acceptable excipients, for the treatment of erectile dysfunction.

In another aspect, the invention deals with pharmaceutical composition comprising a therapeutically effective amount of the said derivatives together with pharmaceutically acceptable excipients for the treatment of clinical conditions presenting vasoconstriction.

In a further aspect, the pharmaceutical compositions according to the invention are suitable for an intracavernosal injection, or in the form of an ointment, a cream or a suspension or a solution or an injectable.

These aspects and preferred embodiments thereof are additionally also defined in the claims.

### DETAILED DESCRIPTION OF THE INVENTION

The inventors of the present application have surprisingly found that some selective 5-HT7 agonists of formula (I) show a vasodilator activity. In particular, these compounds induce a peripheral vasodilatation when administered to a mammal. More specifically, selective 5-HT7 agonists of formula (I) induce penile erection after local injection (intrapenis or intracavernosal injection) or topical administration. The present invention provides an alternative therapy for the treatment of ED with high efficacy and safety. Selective 5-HT7 agonists of formula (I) could represent a promising therapy directed to ED caused by vascular pathologies such as peripheral arterial disease and endothelial dysfunction seen in diabetes mellitus, atherosclerosis, coronary disease and hypertension.

By the term mammal as used herein is meant an individual belonging to the class Mammalia. The invention is particularly useful in the treatment of human patients.

As used herein, the terms "compounds of the invention" and "compound of formula (I)" means pharmaceutically acceptable derivatives thereof and polymorphs, isomers and isotopically labelled variants thereof. Furthermore, the term "compounds of the invention" and "compound of formula (I)" include compounds of formulae (la), (lb) and (lc) and the embodiments thereof disclosed herein.

Thus, in a first aspect, the invention deals with heterocyclyl substituted-phenyl derivatives of a general formula (I) for use as vasodilator agent: 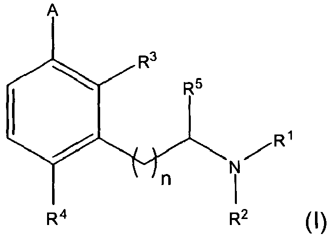 wherein

A is selected from the following group: 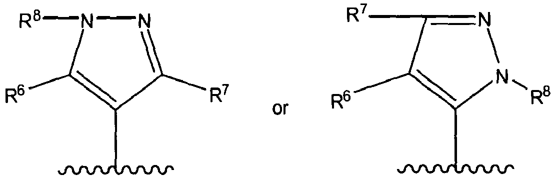 n is selected from 0 or 1,
- R¹ and R² each are independently selected from the group consisting of hydrogen, a linear or branched, saturated or unsaturated, optionally at least mono-substituted aliphatic radical or at least mono-substituted phenyl radical,
- R³ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; and
   R⁵ is hydrogen; or
   R³ and R⁵ together form with the carbon atom to which they are attached, a saturated or unsaturated, optionally at least mono-substituted, cycloalkyl ring with 5 or 6 members; and
   R⁴ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
   or,
- R³ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; and
   R⁴ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; and R⁵ is hydrogen; or
   R⁴ and R⁵ together form with the carbon atom to which they are attached, a saturated or unsaturated, optionally at least mono-substituted, cycloalkyl group ring with 5 or 6 members;
- R⁶ and R⁷ each are independently selected from the group consisting of hydrogen; a phenyl radical, or a linear or branched, saturated or unsaturated, optionally at least mono-substituted aliphatic radical;
- R⁸ is selected from the group consisting of hydrogen; a phenyl radical, or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH,
optionally in form of one of its stereoisomers, preferably enantiomers or diastereomers, its racemate or in form of a mixture of at least two of its stereoisomers, preferably enantiomers or diastereomers, in any mixing ratio, or in form of a salt, preferably a physiologically acceptable salt, more preferably in form of a physiologically acceptable acid addition salt, or a corresponding solvate.

Aliphatic radical, as referred to in the present invention, are optionally mono- or polysubstituted and may be branched or linear, saturated or unsaturated. Aliphatic radicals, as defined in the present invention, include alkyl, alkenyl and alkynyl radicals. Unsaturated aliphatic radicals, as defined in the present invention, include alkenyl and alkynyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl.

In the context of this invention, "alkyl", "alkyl radical" or "alkyl group" is understood as meaning saturated, linear or branched hydrocarbons, which can be unsubstituted or mono-or polysubstituted. Thus alkyl encompasses e.g. -CH₃ and -CH₂-CH₃- In these radicals, C1-2-alkyl represents C1- or C2-alkyl, C1-3-alkyl represents C1-, C2- or C3-alkyl, C1-4-alkyl represents C1-, C2-, C3- or C4-alkyl, C1-5-alkyl represents C1-, C2-, C3-, C4-, or C5-alkyl, C1-6-alkyl represents C1-, C2-, C3-, C4-, C5- or C6-alkyl, C1-7-alkyl represents C1-, C2-, C3-, C4-, C5-, C6- or C7-alkyl, C1-8-alkyl represents C1-, C2-, C3-, C4-, C5-, C6-, C7- or C8-alkyl, C1-10-alkyl represents C1-, C2-, C3-, C4-, C5-, C6-, C7-, C8-, C9- or C10-alkyl and C1-18-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH.

According to the present invention, alkenyl radical means an unsaturated alkyl containing one or more double bonds, like e.g. -CH=CH-CH₃. The alkenyl radicals are preferably vinyl (ethenyl), allyl (2-propenyl).

According to the present invention, alkynyl radical mean an unsaturated alkyl containing one or more triple bonds, like e.g. -CH≡CH-CH₃.

In connection with alkyl or aliphatic radical or group - unless defined otherwise - the term "substituted" in the context of this invention is understood as meaning replacement of at least one hydrogen radical by F, Cl, Br, I, NH₂, SH or OH; within that "monosubstituted" means the substitution of exactly one hydrogen radical, whereas "polysubstituted" means the substitution of more than one hydrogen radical with "polysubstituted"radicals being understood as meaning that the replacement takes place both on different and on the same atoms several times with the same or different substituents, for example three times on the same C atom, as in the case of CF₃, or at different places, as in the case of e.g. -CH(OH)-CH=CH-CHCl₂. Therefore, "optionally at least monsubstituted" means either "not substituted" if the option is not fulfilled, "monosubstituted" or "polysubstituted".

By the term "halogen" is understood F, Cl, Br or I.

In the context of this invention "cycloalkyl radical" or "cycloalkyl group" is understood as meaning saturated and unsaturated (but not fully aromatic) cyclic hydrocarbons (without a heteroatom in the ring), which can be unsubstituted or mono- or polysubstituted. However, mono- or polyunsaturated, preferably monounsaturated, cycloalkyls also in particular fall under the term cycloalkyl as long as the cycloalkyl is not an aromatic system. The cycloalkyl radicals are preferably cyclopentyl, cyclohexyl, tetralinyl, or indanyl.

An "aryl", "aryl radical" or "aryl group" is understood as meaning aromatic ring systems but without heteroatoms even in only one of the rings. Examples are phenyl, naphthyl, fluoranthenyl, or anthracenyl radicals, which can be unsubstituted or monosubstituted or polysubstituted.

The term "salt" is to be understood as meaning any form of the active compound used according to the invention in which it assumes an ionic form or is charged and is coupled with a counter-ion (a cation or anion) or is in solution.

The term "physiologically acceptable salt" means in the context of this invention any salt that is physiologically tolerated (most of the time meaning not being toxic-especially without toxicity caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention (usually a deprotonated acid) as an anion with at least one, preferably inorganic, cation which is physiologically tolerated especially if used on humans and/or mammals. The salts of the alkali metals and alkaline earth metals are particularly preferred, and also those with NH₄, but in particular (mono)- or (di)sodium, (mono)- or (di)potassium, magnesium or calcium salts.

These physiologically acceptable salts can also be formed with anions or acids and in the context of this invention is understood as meaning salts of at least one of the compounds used according to the invention (usually protonated, for example on the nitrogen) as the cation with at least one anion which is physiologically tolerated especially if used on humans and/or mammals. By this is understood in particular, in the context of this invention, the salt formed with a physiologically tolerated acid, that is to say salts of the particular active compound with inorganic or organic acids which are physiologically tolerated especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a preferred embodiment, the invention deals with heterocyclyl substituted-phenyl derivatives of formula (la) for use as vasodilator agent: 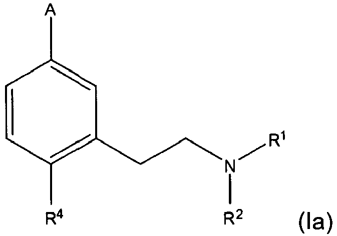 wherein

A is a compound selected from the following group: 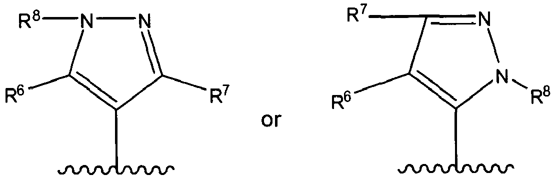
R¹ and R² each are independently selected from the group consisting of hydrogen, a linear or branched, saturated or unsaturated, optionally at least mono-substituted aliphatic radical or a phenyl radical;
R⁴ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ each are independently selected from the group consisting of hydrogen; a phenyl radical, or a linear or branched, saturated or unsaturated, optionally at least mono-substituted aliphatic radical;
R⁸ is selected from the group consisting of hydrogen; a phenyl radical, or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt, more preferably in form of a physiologically acceptable acid addition salt, or a corresponding solvate.

Preferred compounds of formula (la) for use as vasodilators are those wherein A is: 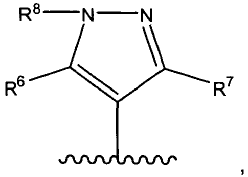 and R⁶, R⁷ and R⁸ have the meaning indicated before for of compounds of formula (la).

In a most preferred embodiment, the invention deals with compounds of formula (la) for use as vasodilators which are:
- 2-(2-(Dimethylamino)ethyl)-4-(1,3,5-trimethyl-1H-pyrazol-4-yl)phenol
- Dimethyl-{2-[3-(1,3,5-trimethyl-1H-pyrazol-4-yl)-phenyl]-ethyl}-amine
- 2-(2-methoxy-5-(1,3,5-trimethyl-1H-pyrazol-4-yl)phenyl)-N,N-dimethylethanamine
- Methyl-{2-[3-(1,3,5-trimethyl-1H-pyrazol-4-yl)-phenyl]-ethyl}-amine,
- Diethyl-{2-[3-(1,3,5-trimethyl-1H-pyrazol-4-yl)-phenyl]-ethyl}-amine,
- Dipropyl-{2-[3-(1,3,5-trimethyl-1H-pyrazol-4-yl)-phenyl]-ethyl}-amine,
- {2-[3-(3,5-Dimethyl-1H-pyrazol-4-yl)-phenyl]-ethyl}-dimethyl-amine,
- {2-[3-(3,5-Dimethyl-1H-pyrazol-4-yl)-phenyl]-ethyl}-methyl-amine,
- {2-[3-(3,5-Dimethyl-1H-pyrazol-4-yl)-phenyl]-ethyl}-diethyl-amine,
- {2-[3-(3,5-Dimethyl-1H-pyrazol-4-yl)-phenyl]-ethyl}-dipropyl-amine,
- Dimethyl-{2-[3-(1-methyl-1H-pyrazol-4-yl)-phenyl]-ethyl}-amine,
- Methyl-{2-[3-(1-methyl-H-pyrazol-4-yi)-phenyl]-ethyl)-amine,
- Diethyl-{2-[3-(1-methyl-1H-pyrazol-4-yl)-phenyl]-ethyl}-amine,
- {2-[3-(1-Methyl-1H-pyrazol-4-yl)-phenyl]-ethyl}-dipropyl-amine,
- [2-(2',6'-Dimethyl-biphenyl-3-yl)-ethyl]-dimethyl-amine,
- [2-(2',6'-Dimethyl-biphenyl-3-yl)-ethyl]-methyl-amine,
- [2-(2',6'-Dimethyl-biphenyl-3-yl)-ethyl]-diethyl-amine,
- [2-(2',6'-Dimethyl-biphenyl-3-yl)-ethyl]-dipropyl-amine,
- [2-(2',6'-Dimethoxy-biphenyl-3-yl)-ethyl]-dimethyl-amine,
- [2-(2',6'-Dimethoxy-biphenyl-3-yl)-ethyl]-methyl-amine,
- [2-(2',6'-Dimethoxy-biphenyl-3-yl)-ethyl]-diethyl-amine,
- [2-(2',6'-Dimethoxy-biphenyl-3-yl)-ethyl]-dipropyl-amine,
- [2-(2'-Methoxy-biphenyl-3-yl)-ethyl]-dimethyl-amine,
- [2-(2'-Methoxy-biphenyl-3-yl)-ethyl]-methyl-amine,
- Diethyl-[2-(2'-methoxy-biphenyl-3-yl)-ethyl]-amine, or
- [2-(2'-Methoxy-biphenyl-3-yl)-ethyl]-dipropyl-amine,
- 2-(2-(Methylamino)ethyl)-4-(1,3,4-trimethyl-1H-pyrazol-5-yl)phenol,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt, more preferably in form of a physiologically acceptable acid addition salt, or a corresponding solvate.

In another embodiment, the invention encompasses heterocyclyl substituted-phenyl derivatives of formula (Ib) for use as vasodilator agents: 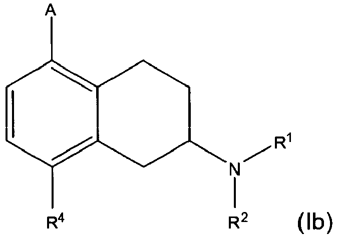 wherein

A is a compound selected from the following group: 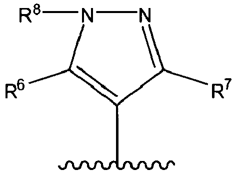
R¹ and R² each are independently selected from the group consisting of hydrogen, a linear or branched, saturated or unsaturated, optionally at least mono-substituted aliphatic radical or at least mono-substituted phenyl radical,
R⁴ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; or
R⁶ and R⁷ each are independently selected from the group consisting of hydrogen; a phenyl radical, or a linear or branched, saturated or unsaturated, optionally at least mono-substituted aliphatic radical;
R⁸ is selected from the group consisting of hydrogen; a phenyl radical, or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt, more preferably in form of a physiologically acceptable acid addition salt, or a corresponding solvate.

A preferred compound of formula (lb) is N,N-dimethyl-5-(1,3,5-trimethyl-1H-pyrazol-4-yl)-1,2,3,4-tetrahydronaphthalen-2-amine.

In a further embodiment, the invention encompasses heterocyclyl substituted-phenyl derivatives of formula (lc) for use as vasodilator agents: 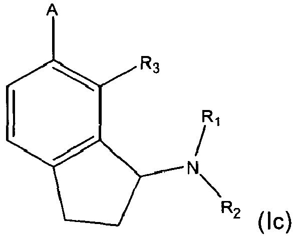 wherein

A is a compound selected from the following group: 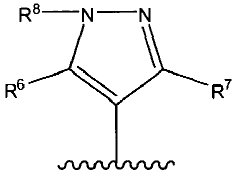
R¹ and R² each are independently selected from the group consisting of hydrogen, a linear or branched, saturated or unsaturated, optionally at least mono-substituted aliphatic radical or at least mono-substituted phenyl radical,
R³ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ each are independently selected from the group consisting of hydrogen; a phenyl radical, or a linear or branched, saturated or unsaturated, optionally at least mono-substituted aliphatic radical;
R⁸ is selected from the group consisting of hydrogen; a phenyl radical, or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH.
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt, more preferably in form of a physiologically acceptable acid addition salt, or a corresponding solvate.

A preferred compound of formula (lc) is N,N-dimethyl-6-(1,3,5-trimethyl-1H-pyrazol-4-yl)-2,3-dihydro-1H-inden-1-amine.

Compounds of formulae (I), (Ia), (Ib) and (Ic) are useful in treating clinical conditions presenting vasoconstriction, including hypertension, pulmonary hypertension, peripheral vascular disease, ischemic conditions including chronic ischemias such as diabetic ischemia, Buerger's Syndrome and Raynaud's Syndrome, and acute ischemias such as those associated with myocardial infarction and stroke. Additionally, the said compounds of the invention, due to their vasodilator properties, are useful in the treatment of penile erectile dysfunction associated with decreased blood flow.

The methods of preparation and synthesis of the aforementioned compounds are described in patent applications WO-2008/077625, WO-2008/095689, WO-2008/116663, WO-2009/003719 and WO-2011/042302.

The amount of the compound of the invention according to formulae (I), (la), (lb) and (Ic) administered should be a therapeutically effective amount where the compound or derivative is used for the treatment of a disease or condition and a prophylactically effective amount where the compound or derivative is used for the prevention of a disease or condition.

The term "therapeutically effective amount" used herein refers to the amount of compound needed to treat or ameliorate a targeted disease or condition. The term "prophylactically effective amount" used herein refers to the amount of compound needed to prevent a targeted disease or condition. The exact dosage will generally be dependent on the patient's status at the time of administration. Factors that may be taken into consideration when determining dosage include the severity of the disease state in the patient, the general health of the patient, the age, weight, gender, diet, time, frequency and route of administration, drug combinations, reaction sensitivities and the patient's tolerance or response to therapy. The precise amount can be determined by routine experimentation, but may ultimately lie with the judgement of the clinician.

The daily dosage for mammals may vary depending on factors that have their basis in the respective species or other factors, such as age, sex, weight or degree of illness and so forth. The daily dosage for humans may preferably be in the range from 1 to 2000, preferably 1 to 1500, more preferably 1 to 1000 milligrams of active substance to be administered during one or several intakes per day.

For pharmaceutical use, the compounds of the invention may be administered as a medicament by several routes, including oral, rectal, sublingual, intracerebroventricular, intrathecal, subcutaneous, intradermal, nasal, intravenous, intramuscular, intraperitoneal, transdermal, transmucuos, local and topical, among others. The compounds of formula (I) should be assessed for their biopharmaceutical properties, such as solubility and solution stability (across pH), permeability, etc., in order to select the most appropriate dosage form and route of administration for treatment of the proposed indication.

The medicament of the present invention may for example be administered as an injectable in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intracavernosally, intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use.

The liquid oral forms for administration may also contain certain additives such as sweeteners, flavoring, preservatives, and emulsifying agents. Non-aqueous liquid compositions for oral administration may also be formulated, containing edible oils.

Such liquid compositions may be conveniently encapsulated in e. g.-, gelatin capsules in a unit dosage amount.

The compositions of the present invention may also be administered topically or via a suppository.

The compounds of the invention may be administered as crystalline or amorphous products. The compounds of the invention may be administered alone or in combination with one or more other compounds of the invention or in combination with one or more other drugs (or as any combination thereof). Generally, they will be administered as a formulation in association with one or more pharmaceutically acceptable excipients. The term "excipient" includes any ingredient other than the compound(s) of the invention which may impart either a functional (e.g drug release rate controlling) and/or a non-functional (e.g. processing aid or diluent) characteristic to the formulations. The choice of excipient will to a large extent depend on factors such as the particular mode of administration, the effect of the excipient on solubility and stability and the nature of the dosage form.

Typical pharmaceutically acceptable excipients include:
- diluents, e.g. lactose, dextrose, sucrose, mannitol, sorbitol, cellulose and/or glycine;
- lubricants, e.g. silica, talcum, stearic acid, its magnesium or calcium salt and/or polyethyleneglycol;
- binders, e.g. magnesium aluminum silicate, starch paste, gelatin, tragacanth, methylcellulose, sodium carboxymethylcellulose and/or polyvinylpyrrolidone;
- disintegrants, e.g. starches, agar, alginic acid or its sodium salt, or effervescent mixtures; and/or
- absorbants, colorants, flavors and/or sweeteners

The concentration of selective 5-HT7 agonists in such a formulation is high enough to permit delivery of a therapeutically effective amount, but not so high as to cause unwanted side effects.

Thus, the invention deals with pharmaceutical compositions comprising a therapeutically effective amount of at least one compound according to formulae (I), (Ia), (Ib) and (Ic) together with pharmaceutically acceptable excipients for the treatment of clinical conditions presenting vasoconstriction.

Also, the invention deals with pharmaceutical compositions comprising a therapeutically effective amount of at least one compound according to formulae (I), (Ia), (Ib) and (Ic) together with pharmaceutically acceptable excipients for the treatment of hypertension.

Additionally, the invention deals with pharmaceutical compositions comprising a therapeutically effective amount of at least one compound according to formulae (I), (Ia), (Ib) and (Ic) together with pharmaceutically acceptable excipients for the treatment of peripheral vascular disease.

Additionally, the invention deals with pharmaceutical compositions comprising a therapeutically effective amount of at least one compound according to formulae (I), (Ia), (Ib) and (Ic) together with pharmaceutically acceptable excipients for the treatment of pulmonary hypertension.

### Oral administration

The compounds of the invention may be administered orally. Oral administration may involve swallowing, so that the compound enters the gastrointestinal tract, and/or buccal, lingual, or sublingual administration by which the compound enters the blood stream directly from the mouth.

Formulations suitable for oral administration include solid plugs, solid microparticulates, semi-solid and liquid (including multiple phases or dispersed systems) such as tablets; soft or hard capsules containing multi- or nano-particulates, liquids (e.g. aqueous solutions), emulsions or powders; lozenges (including liquid-filled); chews; gels; fast dispersing dosage forms; films; ovules; sprays; and buccal/mucoadhesive patches.

Accordingly, in one embodiment, the present invention provides a pharmaceutical composition comprising a therapeutically effective amount of at least one compound of formulae (I) or (Ia) or (Ib) or (Ic) together with pharmaceutically acceptable excipients for oral administration.

### Transdermal and topical administration

Suitable formulations for transdermal application include a therapeutically effective amount of a compound of the invention with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Additionally, suitable preparations comprising the compounds of the invention are external topical formulations to be administered to a penile region, which can be absorbed favourably through the skin or the mucous membrane of a glans and a penis and promotes blood-flow in a glans and a penis, induces penile erection and, at the same time, provides excellent moisturizing effect and reducing effect against frictional resistance.

Most common topical formulations are creams, ointments, pastes, lotions and gels. A cream is an emulsion of water and oil and it can be classified as oil in water (o/w) or water in oil (w/o) and they usually comprise emulsifiers and preservatives. An ointment is a semi-solid preparation of hydrocarbons (petrolatum, mineral oil, paraffins, synthetic hydrocarbons) and which usually has a strong emollient effect useful in dry skin conditions. A paste is a mixture of powder and ointment (e.g. zinc oxide 20% paste). The addition of powder improves the porosity (breathability) and the consistency of the preparation. A lotion can include any liquid preparation in which inert or active medications are suspended or dissolved. A gel is a transparent preparation containing cellulose ethers or carbomer in water or a water-alcohol mixture.

Topical drug formulations should comprise ointment bases which function as protectives and emollients for the skin. They should be compatible with the skin, stable, permanent, smooth and pliable, nonirritating, nonsensitizing, inert and readily able to release its incorporated medication. Examples of ointment bases as well as other substances used together with them to contribute with some pharmaceutical property are: oleaginous ointment bases (e.g. waxes, oleic acid, oils, paraffin, petrolatum, spermaceti, starch glicerite), absorbent or emulsifiable ointment bases (e.g. anhydrous lanolin, hydrophilic petrolatum), emulsion ointment bases (e.g. cetyl alcohol, cetyl esters, glyceryl monoestearate, lanolin, stearic acid), water-soluble ointment bases (e.g. glycol esters, polysorbates, polyethylene glycols, polyoxy estearates). Skin penetration enhancers which serve to improve drug absorption across the skin are also comprised in topical formulations. Ideal penetration enhancers should not affect available dosage forms (e.g. cream or gel) or cosmetic quality of the topical composition.

Accordingly, in one embodiment, the present invention provides a pharmaceutical composition comprising a therapeutically effective amount of at least one compound of formulae (I) or (Ia) or (Ib) or (Ic) together with pharmaceutically acceptable excipients for topical administration.

### Inhalation and intranasal administration

The compounds of the invention can be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler, as an aerosol spray from a pressurised container, pump, spray, atomiser (preferably an atomiser using electrohydrodynamics to produce a fine mist), or nebuliser, with or without the use of a suitable propellant, such as 1, 1, 1,2-tetrafiuoroethane or 1, 1, 1,2,3,3,3-heptafluoropropane, or as nasal drops. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenization or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as /-leucine, mannitol or magnesium stearate. The lactose may be anhydrous or in the form of the monohydrate, preferably the latter. Other suitable excipients include dextran, glucose, maltose, sorbitol, xylitol, fructose, sucrose and trehalose.

Formulations for inhaled/intranasal administration may be formulated to be immediate and/or modified release using, for example, poly(lactic-c -glycolic acid) (PGLA). Modified release formulations include delayed-, sustained-, pulsed-, controlled-, targeted and programmed release.

Accordingly, in one embodiment, the present invention provides a pharmaceutical composition comprising a therapeutically effective amount of at least one compound of formulae (I) or (Ia) or (Ib) or (Ic) together with pharmaceutically acceptable excipients for intranasal administration.

### Intracavernosal injection or local intrapenis injection

The term "intracavernosal" or "intracavernosum" as used herein refers to a mode of drug administration and involves injection into one or both corpora of the corpora cavernosal tissue of the penis. "Carriers" or "vehicles" as used herein refer to carrier materials suitable for local drug administration. Carriers and vehicles useful herein include any such materials known in the art which is nontoxic and does not interact with other components of the composition in a deleterious manner.

Intracavernosal injection can be carried out by use of a syringe or other suitable device. An example of a hypodermic syringe useful herein, that can be used for simultaneous injection in both corpora is described in US 4,127,118. The injection is made on the dorsum of the penis by placement of the needle to the side of each dorsal vein and inserting deep into the corpora.

For intracavernosal injection, the active agent to be administered is incorporated into a sterile liquid preparation, typically a solution or suspension in an aqueous or oleaginous medium. The solution or suspension may be formulated according to techniques known in the art using suitable carriers, dispersants, wetting agents, diluents, suspending agents or the like. Among the acceptable vehicles and solvents that may be employed are water, isotonic saline, vegetable oil, fatty esters and polyols.

Accordingly, in one embodiment, the present invention provides a pharmaceutical composition comprising a therapeutically effective amount of at least one compound of formulae (I) or (Ia) or (Ib) or (Ic) together with pharmaceutically acceptable excipients for intracavernosal administration.

Selective 5-HT7 receptor agonists, such as compounds 1 and 2, after local injection (animal studies) cause penile erection in the absence of sexual stimulation. These observations indicate that the compounds of the present invention result in a new and novel mechanism of treating ED.

The following examples are merely illustrative of certain embodiments of the invention and do not limit it.

### EXAMPLES

### Example 1: 2-(2-(Dimethylamino)ethyl)-4-(1,3,5-trimethyl-1H-pyrazol-4-yl)phenol (Compound 1)

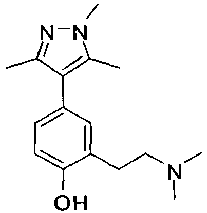

2-(2-methoxy-5-(1,3,5-trimethyl-1H-pyrazol-4-yl)phenyl)-N,N-dimethylethanamine(2.64 mmol) was dissolved, under argon atmosphere, in 70 mL of CH₂Cl₂ and cooled to 0°C. Boron tribromide (13.22 mmol) was slowly added and the reaction mixture was allowed to reach room temperature and stirred for 6 hours. The reaction mixture was added over water and ice and it was stirred overnight. Then, it was basified until pH= 8-9 and the product was extracted with Et₂O. The organic phase was dried with anhydrous Na₂SO₄, filtered and evaporated to dryness to give the crude product 2-(2-(dimethylamino)ethyl)-4-(1,3,5-trimethyl-1H-pyrazol-4-yl)phenol. The crude was purified by flash column chromatography (CH₂Cl₂:MeOH as eluents) to yield the title compound 1 (84%). MS [MH]⁺= 274.0.

### Preparation of the hydrochloride salt of compound 1

2-(2-(Dimethylamino)ethyl)-4-(1,3,5-trimethyl-1H-pyrazol-4-yl)phenol (1.53 mmol) was diluted in 40 mL of AcOEt and 0.69 mL of hydrogen chloride 2 M in Et₂O (1.37 mmol) were added. The resulting precipitate was filtered and washed with AcOEt (75%).

MS [MH]⁺= 274.0.
¹H NMR (300 MHz, Methanol-d4) δ ppm 7.06 (d, J=2.2 Hz, 1H), 7.01 (dd, J=8.2, 2.2 Hz, 1H), 6.88 (d, J=8.2 Hz, 1H), 3.74 (s, 3H), 3.45-3.35 (m, 2H), 3.07 (dd, J=9.5, 6.3 Hz, 2H), 2.21 (s, 3H), 2.15 (s, 3H).

### Example 2: Dimethyl-{2-[3-(1,3,5-trimethyl-1H-pyrazol-4-yl)-phenyl]-ethyl}-amine (Compound 2)

Prepared according to example 1 of international patent application WO-2008/077625.

### Example 3: N,N-dimethyl-5-(1,3,5-trimethyl-1H-pyrazol-4-yl)-1,2,3,4-tetrahydronaphthalen-2-amine (Compound 3)

Prepared according to International patent application WO-2008/116663.

### Example 4: N,N-dimethyl-6-(1,3,5-trimethyl-1H-pyrazol-4-yl)-2,3-dihydro-1H-inden-1-amine (Compound 4)

Prepared according to Example 2 of international paent application WO-2009/003719.

### Example 5: 2-(2-methoxy-5-(1,3,5-trimethyl-1H-pyrazol-4-yl)phenyl)-N,N-dimethylethan-amine (Compound 5)

Prepared according to Example 45 of international patent application WO-2008/077625.

### Example 6: Effects of 5-HT7 receptor agonists on peripheral vasodilation (skin color score)

### Material and Methods

### Animals

Male Wistar rats aged from 5 to 6 weeks old (100 - 150 g) were used in these studies. Animals were housed in groups of five, provided with food and water *ad libitum* and kept in controlled laboratory conditions with the temperature maintained at 22 ± 2 °C and light in 12 h cycles (on at 06:00 h and off at 18:00 h). Experiments were carried out in a soundproof and air-regulated experimental room. The number of rats ranged from 8 to 10 per group in each individual experiment.

### Drugs

Drugs used for treatments were: Compound 1, Compound 2, Compound 3, Compound 4 and Compound 5. Compounds were dissolved in aqueous solution containing 0.5% hydroxypropyl methyl cellulose (HPMC). All the compounds and vehicle (0.5% HPMC) were administered in a volume of 10 ml/kg through the intraperitoneal (i.p.) route.

### Experimental Procedure

Male Wistar rats received i.p. injections of test substances and peripheral vasodilation was quantified (skin color score) from 30-180 min after drug administration, by giving vasodilation score (skin rose color score). The Baseline was determined based on vehicle injection. Data are expressed as Mean±SEM.

Peripheral vasodilation (using skin color as a qualitative index) was assessed for 3 hours (30, 60, 120 and 180 min) after dosing with vehicle (0.5% HPMC), Compound 1, Compound 2, Compound 3, Compound 4 or Compound 5.

Skin color on rats was measured by using a scale from 0-3 with:
0 = white (normal vasodilation)
1 = pink (slight vasodilation)
2 = red (moderate vasodilation)
3 = dark red (intense vasodilation)

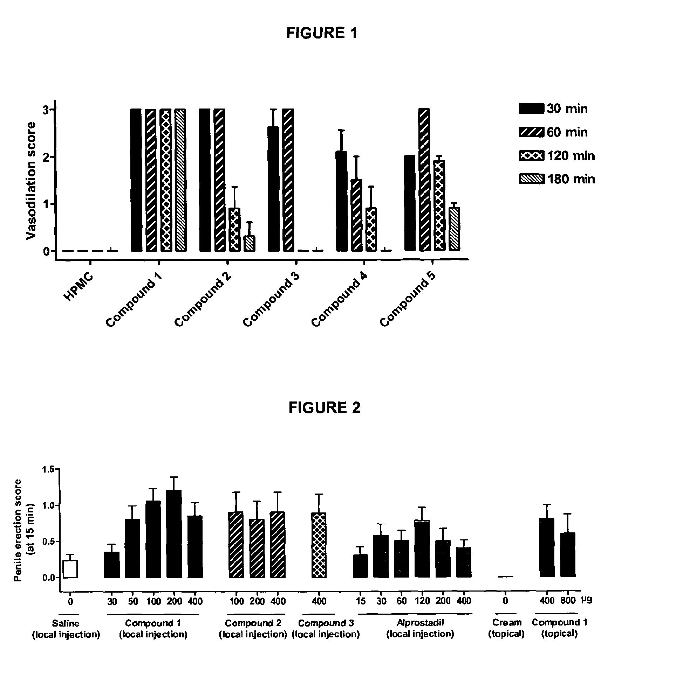

Figure 1 shows the results of the said peripheral vasodilation test.

The test results refer to:
Vehicle [0.5% HPMC, 10 ml/kg, i.p.]
Compound 1 [40 mg/kg, i.p.]
Compound 2 [40 mg/kg, i.p.]
Compound 3 [40 mg/kg, i.p.]
Compound 4 [40 mg/kg, i.p.]
Compound 5 [40 mg/kg, i.p.]

Compounds according to the invention show a high effect on vasodilation of blood vessels.

### Example 7: Effects of Compounds 1 and 2 on penile erection in conscious rats

### Material and Methods

### Animals

Male Wistar rats aged from 8 to 10 weeks old (250 - 320 g) were used in these studies. Animals were housed in groups of five, provided with food and water *ad libitum* and kept in controlled laboratory conditions with the temperature maintained at 22 ± 2 °C and light in 12 h cycles (on at 06:00 h and off at 18:00 h). Experiments were carried out in a soundproof and air-regulated experimental room. The number of rats ranged from 5 to 34 per group in each individual experiment.

### Drugs

Drugs used for treatments were: Compound 1, Compound 2, Compound 3 and Alprostadil.

### Experimental Procedure

Male Wistar rats received local injections (intrapenis injections) or topical administrations of test substances and penile erection was quantified (penile erection score) from 15 to 30 min after local drug injection and from 5 to 60 min after topical drug administration. The magnitude of penile erection is determined by penile erection score. The Baseline was determined based on vehicle injection.

For local injection (intrapenis injection), compounds were dissolved in physiological saline (aqueous solution of 0.9% NaCl). All the compounds and vehicle (0.9% physiological saline) were administered in a volume of 50 µl.

For topical administration, compounds were dissolved in a commercial cream (Eucerinum® Base o/w. Composition: Aqua, Isopropyl Palmitate, Glyceryl Stearate, Paraffinum Liquidum, Glycerin, Stearth-21, Microcrystalline wax, Steareth-2, PEG-8, Distearate, Dimethicone, Benzyl Alcohol, Lanolin Alcohol, Phenoxyethanol). All the compounds and vehicle were administered in a volume of 20 µl by topical application directly on the exposed penis, with a gentle massage until absorption of the cream.

For evaluating the penile erection after both types of administration, a qualitative score method was used, where 0 = no effect, 1 = slight effect, 2 = some effect without exposure of the penis, and 3 = clear effect with exposure of the penis. The reading times for the local injection were 15 and 30 minutes, and for the topical application were 5, 15, 30, and 60 minutes.

Figure 2 shows the results of the said penile erection test: data obtained at 15 min after drug administration. Data are expressed as Mean±SEM.

The test results refer to:
Vehicle [saline, 50 µl, local injection]
Vehicle [cream, 20 µl, topical]
Compound 1 [30, 50, 100, 200 and 400 µg/50 µl, local injection]
Compound 1 [400 and 800 µg/20 µl, topical]
Compound 2 [100, 200 and 400 µg/50 µl, local injection]
Compound 3 [400 µg/50 µl, local injection]
Alprostadil [15, 30, 60, 120, 200 and 400 µ/50 µl, local injection]

Compounds according to the invention show a high effect on penile erection.

## Claims

1. Compounds of general formula (I) for use as vasodilator agent: 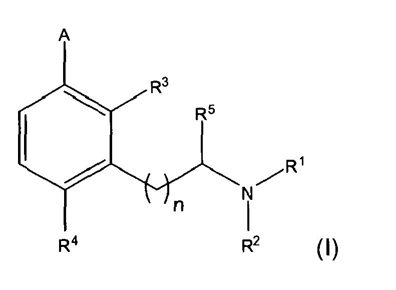 wherein
A is a compound selected from the following group: 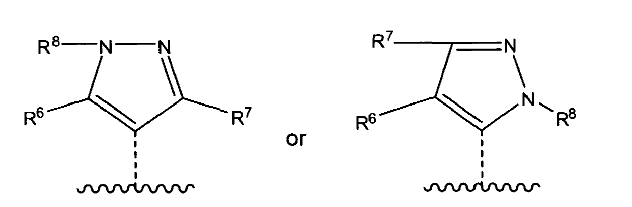
n is selected from 0 or 1,
• R¹ and R² each are independently selected from the group consisting of hydrogen, a linear or branched, saturated or unsaturated, optionally at least mono-substituted aliphatic radical or at least mono-substituted phenyl radical,
• R³ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; and R⁵ is hydrogen; or
R³ and R⁵ together form with the carbon atom to which they are attached, a saturated or unsaturated, optionally at least mono-substituted, cycloalkyl ring with 5 or 6 members; and
R⁴ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
**or**
. R³ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; and
R⁴ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; and R⁵ is hydrogen; or
R⁴ and R⁵ together form with the carbon atom to which they are attached, a saturated or unsaturated, optionally at least mono-substituted, cycloalkyl group ring with 5 or 6 members;
• R⁶ and R⁷ each are independently selected from the group consisting of hydrogen; a phenyl radical, or a linear or branched, saturated or unsaturated, optionally at least mono-substituted aliphatic radical;
• R⁸ is selected from the group consisting of hydrogen; a phenyl radical, or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt, more preferably in form of a physiologically acceptable acid addition salt, or a corresponding solvate.

2. Compounds of formula (Ia) according to claim 1 for use as vasodilator agent: 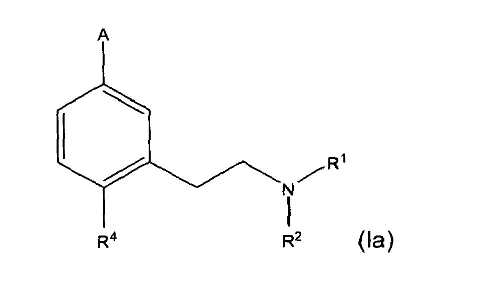 wherein
A is a compound selected from the following group: 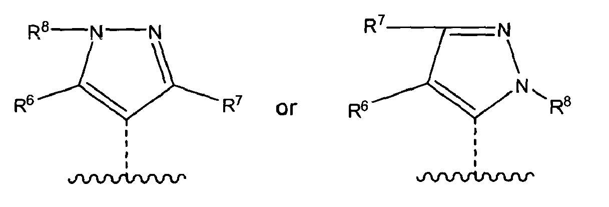
R¹ and R² each are independently selected from the group consisting of hydrogen, a linear or branched, saturated or unsaturated, optionally at least mono-substituted aliphatic radical or a phenyl radical;
R⁴ is selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ each are independently selected from the group consisting of hydrogen; a phenyl radical, or a linear or branched, saturated or unsaturated, optionally at least mono-substituted aliphatic radical;
R⁸ is selected from the group consisting of hydrogen; a phenyl radical, or an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt, more preferably in form of a physiologically acceptable acid addition salt, or a corresponding solvate.

3. Compounds according to claim 2 for use as vasodilator agent, wherein group A is 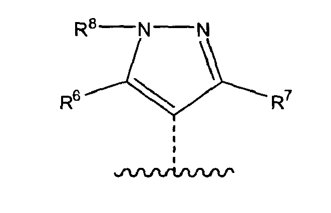 and R⁹, R⁷ and R⁸ have the meaning according to claim 1,
optionally in form of one of its stereoisomers, preferably enantiomers or diastereomers, its racemate or in form of a mixture of at least two of its stereoisomers in any mixing ratio, or a physiologically acceptable salt thereof, or a solvate.

4. Compounds according to any of claims 1 and 2 for use as vasodilator agent, selected from
• 2-(2-(Dimethylamino)ethyl)-4-(1,3,5-trimethyl-1H-pyrazol-4-yl)phenol
• Dimethyl-{2-[3-(1,3,5-trimethyl-1H-pyrazol-4-yl)-phenyl]-ethyl}-amine
• 2-(2-methoxy-5-(1,3,5-trimethyl-1H-pyrazol-4-yl)phenyl)-N,N-dimethylethanamine
• Methyl-{2-[3-(1,3,5-trimethyl-1H-pyrazol-4-yl)-phenyl]-ethyl}-amine,
• Diethyl-{2-[3-(1,3,5-trimethyll-1H-pyrazol-4-yl)-phenyl]-ethyl}-amine,
• Dipropyl-{2-[3-(1,3,5-trimethyl-1H-pyrazol-4-yl)-phenyl]-ethyl}-amine,
• {2-[3-(3,5-Dimethyl-1H-pyrazol-4-yl)-phenyl]-ethyl}-dimethyl-amine,
• {2-[3-(3,5-Dimethyl-1H-pyrazol-4-yl)-phenyl]-ethyl}-methyl-amine,
• {2-[3-(3,5-Dimethyl-1H-pyrazol-4-yl)-phenyl]-ethyl}-diethyl-amine,
• {2-[3-(3,5-Dimethyl-1H-pyrazol-4-yl)-phenyl]-ethyl}-dipropyl-amine,
• Dimethyl-{2-[3-(1-methyl-1H-pyrazol-4-yl)-phenyl]-ethyl}-amine,
• Methyl-{2-[3-(1-methyl-1H-pyrazol-4-yl)-phenyl]-ethyl}-amine,
• Diethyl-{2-[3-(1-methyl-1H-pyrazol-4-yl)-phenyl]-ethyl}-amine,
• {2-[3-(1-Methyl-1H-pyrazol-4-yl)-phenyl]-ethyl}-dipropyl-amine,
• [2-(2',6'-Dimethyl-biphenyl-3-yl)-ethyl]-dimethyl-amine,
• (2-(2',6'-Dimethyl-biphenyl-3-yl)-ethyl]-methyl-amine,
• [2-(2',6'-Dimethyl-biphenyl-3-yl)-ethyl]-diethyl-amine,
• [2-(2',6'-Dimethyl-biphenyl-3-yl)-ethyl]-dipropyl-amine,
• [2-(2',6'-Dimethoxy-biphenyl-3-yl)-ethyl]-dimethyl-amine,
• [2-(2',6'-Dimethoxy-biphenyl-3-yl)-ethyl]-methyl-amine,
• [2-(2',6'-Dimethoxy-biphenyl-3-yl)-ethyl]-diethyl-amine,
• [2-(2',6'-Dimethoxy-biphenyl-3-yl)-ethyl]-dipropyl-amine,
• [2-(2'-Methoxy-biphenyl-3-yl)-ethyl]-dimethyl-amine,
• [2-(2'-Methoxy-biphenyl-3-yl)-ethyl]-methyl-amine,
• Diethyl-[2-(2'-methoxy-biphenyl-3-yl)-ethyl]-amine, or
• [2-(2'-Methoxy-biphenyl-3-yl)-ethyl]-dipropyl-amine,
• 2-(2-(Methylamino)ethyl)-4-(1,3,4-trimethyl-1H-pyrazol-5-yl)phenol,
optionally in form of one of its stereoisomers, preferably enantiomers or diastereomers, its racemate or in form of a mixture of at least two of its stereoisomers in any mixing ratio, or a physiologically acceptable salt thereof, or a solvate.

5. Compounds according to any of claims 1 to 4 for the treatment of erectile dysfunction.

6. Compounds according to claim 5 for the treatment of erectlie dysfunction caused by peripheral arterial disease and/or endothelial dysfunction seen in diabetes mellitus and/or atherosclerosis and/or coronary disease and/or hypertension.

7. Compounds according to claims 1 to 4 for the treatment of clinical conditions presenting vasoconstriction.

8. Compounds according to claims 1 to 4 for the treatment of hypertension.

9. Compounds according to claims 1 to 4 for the treatment of peripheral vascular disease.

10. Compounds according to any of claims 1 to 4 for the treatment of pulmonary arterial hypertension.

11. Pharmaceutical compositions comprising a therapeutically effective amount of at least one compound according to any of claims 1 to 4 together with pharmaceutically acceptable excipients for the treatment of erectile dysfunction.

12. Pharmaceutical compositions comprising a therapeutically effective amount of at least one compound according to any of claims 1 to 4 together with pharmaceutically acceptable excipients for the treatment of clinical conditions presenting vasoconstriction.

13. Pharmaceutical compositions according to claim 11 which is an injectable.

14. Pharmaceutical composition according to claim 13 which is suitable for an intracavernosal injection.

15. Pharmaceutical composition according to claim 11 which is an ointment or a cream.
